# EUROPEAN PATENT SPECIFICATION

(11) **EP 0 738 163 B1**
(45) Date of publication and mention of the grant of the patent: **05.02.2003**
(21) Application number: 95906630.9
(22) Date of filing: 12.12.1994
(51) Int. Cl.: A61M 5/142, A61M 39/24, A61M 5/168

(54) **MEDICAL INFUSION APPARATUS INCLUDING SAFETY VALVE**
MEDIZINISCHE INFUSIONSVORRICHTUNG MIT SICHERHEITSVENTIL
APPAREIL POUR PERFUSIONS A SOUPAPE DE SURETE

(30) Priority: 13.12.1993 IL 10800493; 03.07.1994 IL 11019894
(43) Date of publication of application: 23.10.1996
(73) Proprietor: MIGADA, INC., Englewood Cliffs, NJ 07632 (US)
(72) Inventor: SHEMESH, Eli, 77700 Ashdod (IL); BARAK, Swi, 38900 Caesaria (IL); RAZ, Haim, 76572 Rehovot (IL)
(74) Representative: Ebner von Eschenbach, Jennifer
(86) International application number: US9414334
(87) International publication number: WO95016480

(56) References cited:
- EP-A- 0 273 714
- EP-A- 0 398 583
- DE-A- 3 035 748
- DE-C- 4 126 088
- FR-A- 2 338 710
- US-A- 4 722 731
- US-A- 5 211 201
- US-A- 5 356 379

## Description

The present invention relates to medical infusion apparatus such as used for administering an infusion liquid intravenously to a subject. The invention relates in particular to a safety valve included in such infusion apparatus.

For many years, infusion liquids have been administered to subjects intravenously by gravity feeding the infusion liquid from a container, e.g., a plastic bag, supported above the subject. More recently, such infusion apparatus have included pump, particularly peristaltic pumps, which provide better control of the infusion rate. The use of such pumps, however, has created a dangerous situation which can occur before the pump has been connected into the infusion tubing or after it has been disconnected therefrom. Thus, in either of such situations, the attendant may accidentally leave the infusion tubing connected to the patient and not be aware that the patient is receiving infusion liquid by gravity feed from the infusion bag. This can be harmful, or even fatal, to the patient. Many pump constructions for infusion apparatus are provided with special protective arrangements to preclude this possible danger, but the danger still exists when infusion apparatus is used with a pump not provided with such special protection.

An object of the present invention is provide medical infusion apparatus that includes protection against this possible danger even when the apparatus is used with a pump not provided with such a protective feature. Another object of the invention is to provide medical infusion apparatus which can be conveniently primed, i.e., purged of all air in the tubing, before the apparatus is connected to the patient.

DE 41 26 088 C1 discloses a valve for a hose for an infusion apparatus in which the valve is normally closed but is opened when the pump is operated. The valve is also manually deformable to open the valve. The valve construction requires a multitude of parts and utilizes a specially configured outlet section with a flexible lip to seal against an inserted plug to control flow of liquid past the valve.

DE 30 35 748 shows an infusion apparatus having a normally closed safety valve openable when the pump is operated.

EP 0 273 714 shows a venting apparatus with a pump and a normally closed valve when the pump is operated.

USP 4,722,731 shows an air check valve for an IV system without a pump. The check valve opens automatically when fluid pressure exceeds a predetermined value.

According to the present invention, there is provided medical infusion apparatus, comprising: a container for a liquid to be infused; tubing leading from the container to the subject to receive an infusion; a pump for pumping the infusion liquid via the tubing to the subject; and a safety valve connected to the tubing between the pump and the subject; the safety valve being normally closed to prevent free-flow of the liquid from the container but being automatically opened when a predetermined minimum pressure is applied to the liquid by the pump, the safety valve being characterized by comprising a cannula (20) having an axial passageway (21) from one end of the cannula but closed at the opposite end, and a radial bore (23) adjacent said opposite end leading from said axial passageway to the outer surface of the cannula; and an elastomeric sleeve (24) around the cannula and overlying and normally closing said radial bore, said safety valve also being manually deformable e.g., by squeezing, bending or twisting.

Several embodiments of the invention are described below for purposes of example.

Medical infusion apparatus equipped with such a safety valve normally blocks the flow of the infusion liquid, thereby preventing infusion liquid from being gravity-fed to the patient should the pump be disconnected. However, such a safety valve is automatically opened by a predetermined minimum liquid pressure as applied by the pump. It may also be manually opened by manually deforming (e.g., squeezing, bending or twisting) it to permit priming the apparatus. Such apparatus thus provides protection against the above-mentioned danger of inadvertently feeding infusion liquid to the patient when the pump is disconnected, and also enables the apparatus to be primed in a convenient manner.
Fig. 1 pictorially illustrates one form of medical infusion apparatus in accordance with the present invention;
Fig. 2 is a longitudinal sectional view illustrating one form of safety valve that may be used with the apparatus in accordance with the present invention;
Figs. 3, 4 and 5 are end views illustrating the safety valve of Fig. 2, respectively, without the elastomeric sleeve, with the elastomeric sleeve in its closed condition, and with the elastomeric sleeve in its open condition;
Fig. 6 is a longitudinal sectional view illustrating a modification in the construction of the cannula in the safety valve of Figs. 2-5;
Fig. 7 is a side view illustrating the safety valve of Fig. 6 assembled with the elastomeric sleeve;

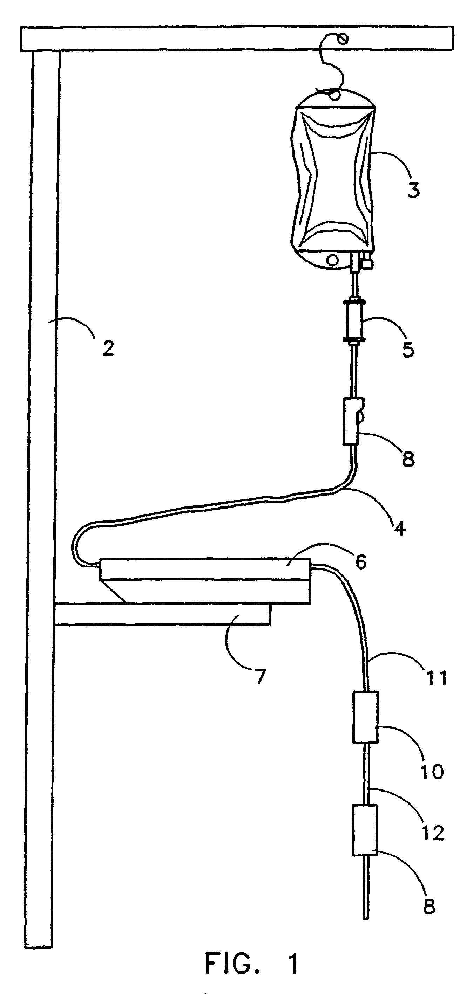

The infusion apparatus illustrated in Fig. 1 includes a holder 2 for holding a container 3, such as a plastic bag, for the infusion liquid at a height above that of the subject to receive the infusion. The infusion is fed via tubing 4 connected at one end to the infusion container 3, and including a needle or luer connector (not shown) at the opposite end for administering the infusion liquid intravenously to the subject. The illustrated apparatus further includes a drip chamber 5, a pump (e.g., a peristaltic pump) 6 supported on a support 7, and optionally, roller clamps 8 that may be used for controlling the flow of the infusion liquid to the subject.

As indicated earlier, there is a danger that, either before pump 6 is connected to the infusion tubing 4 or after it has been disconnected, the attendant will not be aware that the infusion tubing is still attached to the subject so that the subject still receives infusion liquid by gravity feed from the infusion container 3. When the pump 6 is not connected, the attendant should shut-off the flow of the infusion liquid by a roller clamp 8, but the attendant may inadvertently not do this and at the same time not be aware that the subject is receiving infusion liquid by gravity feed. As indicated above, this can be extremely harmful and possibly even fatal, to the patient.

To reduce or eliminate this danger, the infusion tubing 4 illustrated in Fig. 1 further includes a safety valve, generally designated 10. Safety valve 10 is connected by tubing 11 on one side to the pump 6, and on the opposite side to tubing 12 leading to the patient. The safety valve is normally closed so that it automatically blocks the flow of the infusion liquid through the tubing 12; but automatically opens when a predetermined liquid pressure is applied by the infusion liquid to the safety valve. This predetermined pressure is larger than that of the pressure head but smaller than that produced by pump 6 so that the safety valve is automatically closed when the pump is not connected, but automatically opens as soon as the pump is connected and operated.

Preferably, safety valve 10 should prevent free flow at pressures that are less than 0.2 bar (atm), and should allow a maximum pressure drop of approximately 0.4 bar (atm). Pump 6 may provide a maximum pressure of 0.5 bar (atm).

As will be described below, safety valve 10 may also be manually opened by manually squeezing it to permit priming the apparatus, i.e., purging it of air.

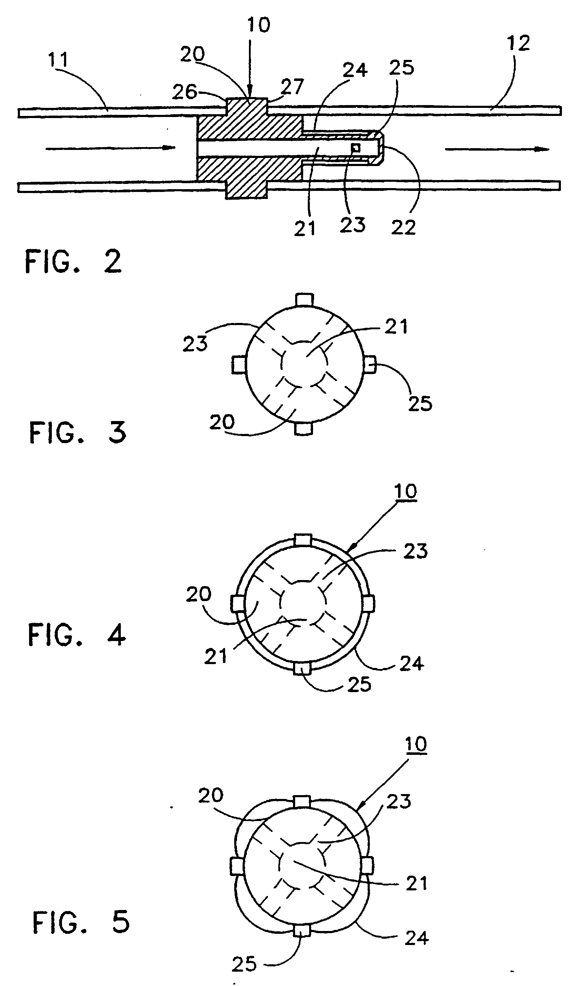

Figs. 2-7 illustrate examples of constructions that the safety valve 10 may have in order to perform the above functions.

Safety valve 10 illustrated in Figs. 2-5 includes a cannula 20 having an axial passageway 21 starting from one end and closed at the opposite end 22. Cannula 20 is further formed with four radial bores 23 adjacent the closed end 22 and leading from the axial passageway 21 to the outer surface of the cannula. An elastomeric sleeve 24 is applied around the cannula overlying the radial bores 23. The outer surface of the cannula is further formed with a ribbed formation 25 including four circumferentially-spaced ribs, for locating the elastomeric sleeve 24 and preventing it from slipping over the end of the cannula.

One end of cannula 20 is formed with an annular seat 26 for receiving tube 11, and its opposite end is formed with another annular seat 27 for receiving tube 12, whereby the cannula connects together the two tubes 11, 12, with the safety valve in between.

It will thus be seen that the radial bores 23 are normally closed by the elastomeric sleeve 24, thereby blocking the flow from tube 11 to tube 12. However, when a sufficient pressure is applied by the pump 6 (Fig. 1), sleeve 24 deforms, as shown in Fig. 5, to permit fluid flow from tube 11 to tube 12 via passageway 21 and the opened radial bores 23.

It will also be seen that the cannula 20 and the overlying sleeve 24 may be squeezed together to open the radial bores 23 in order to permit manual priming of the apparatus before connecting it to the pump. Thus, squeezing the opposite sides of the sleeve 24 will deform it into an elliptical shape and will cause the portions of the sleeve not engaged by the user's fingers to move outwardly away from the outer surface of the cannula to thereby uncover the respective radial bores 23. This opening of the radial bores to permit manual priming of the apparatus would be further enhanced by making the cannula 20 also deformable by this squeezing pressure.

Preferably, the inner faces of the cannula 20 and the elastomeric sleeve 24 are lubricated with a silicone oil. Medically approved oil for this purpose is available from Dow Corning.

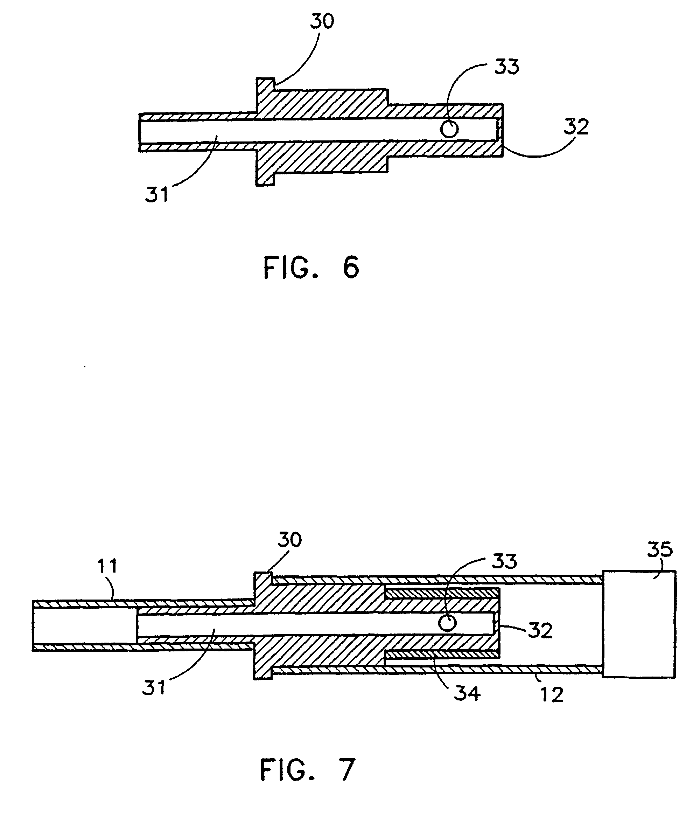

Fig. 6 illustrates a modified construction of the cannula which may be used as the safety valve. In this case, the cannula, generally designated 30, is also formed with an actual passageway 31 closed at one end 32 but communicating with four radial bores 33 adjacent to the closed end and leading to the outer surface of the cannula. As shown by Fig. 7, the radial bores 33 are closed by an elastomeric sleeve 34 connected at one end to one part of a luer lock 35, which is matable with the other part of a luer lock (not shown) to which the other tube (12, Fig. 2) is connected.

## Claims

1. Medical infusion apparatus, comprising: a container (3) for a liquid to be infused; tubing (4) leading from the container to the subject to receive an infusion; a pump (6) for pumping the infusion liquid via said tubing to the subject; and a safety valve (10) connected in said tubing between said pump and the subject; said safety valve being normally closed to prevent free-flow of the liquid from the container but being automatically opened when a predetermined minimum pressure is applied to the liquid by said pump; **characterized in that** said safety valve comprises a cannula (20) having an axial passageway (21) from one end of the cannula but closed at the opposite end, and a radial bore (23) adjacent said opposite end leading from said axial passageway to the outer surface of the cannula; and an elastomeric sleeve (24) around the cannula and overlying and normally closing said radial bore; said safety valve also being manually deformable e.g. by squeezing, bending or twisting to open the valve and to permit priming of the apparatus by purging it of air.

2. The apparatus according to claim 1, **characterized in that** said cannula includes an annular seat (26, 27) at both ends for receiving said tubing in order to connect two sections (11, 12) of said tubing together with the safety valve in between.

3. The apparatus according to claim 1, **characterized in that** the outer surface of said opposite end of the cannula is formed with a ribbed formation (25) for locating said elastomeric sleeve.

4. The apparatus according to claim 1, **characterized in that** a lubricant is applied between the outer surface of the cannula and the inner surface of the elastomeric sleeve.

## Patentansprüche

1. Medizinischer Infusionsapparat, umfassend: einen Behälter (3) für eine zu infundierende Flüssigkeit; Schlauchleitung (4), die von dem Behälter zu dem Patienten führt, der eine Infusion erhält; eine Pumpe (6) zum Pumpen der Infusionsflüssigkeit über die Schlauchleitung zu dem Patienten; und ein Sicherheitsventil (10), das mit der Schlauchleitung verbunden ist, und sich zwischen der Pumpe und dem Patienten befindet; wobei das Sicherheitsventil normalerweise geschlossen ist, um ein freies Strömen der Flüssigkeit von dem Behälter zu verhindern, das jedoch automatisch geöffnet wird, wenn ein vorbestimmter Mindestdruck von der Pumpe auf die Flüssigkeit gegeben wird; **dadurch gekennzeichnet, dass** das Sicherheitsventil eine Kanüle 20 aufweist, die von dem einen Ende der Kanüle einen axialen Durchgang (21) hat und an dem gegenüberliegenden Ende geschlossen ist, sowie eine radiale Bohrung (23), angrenzend an dem gegenüberliegenden Ende hat, die von dem axialen Durchgang zu der Außenseite der Kanüle führt; sowie eine elastomere Manschette (24) um die Kanüle und über der radialen Bohrung sitzt und diese normalerweise geschlossen hält; wobei das Sicherheitsventil auch manuell deformiert werden kann, z.B. durch Drücken, Biegen oder Verdrillen, um das Ventil zu öffnen und ein Ansaugen des Apparates zu ermöglichen, indem dieser mit Luft gespült wird.

2. Apparat nach Anspruch 1, **dadurch gekennzeichnet, dass** in die Kanüle eine kreisrunde Befestigungsfläche (26, 27) an beiden Enden zur Aufnahme der Schlauchleitung einbezogen ist, um die zwei Abschnitte (11, 12) der Schlauchleitung zusammen mit dem Sicherheitsventil dazwischen zu verbinden.

3. Apparat nach Anspruch 1, **dadurch gekennzeichnet, dass** die Außenseite des gegenüberliegenden Endes der Kanüle mit einer Rippenbildung (25) zur Anbringung der elastomeren Muffe versehen ist.

4. Apparat nach Anspruch 1, **dadurch gekennzeichnet, dass** zwischen der Außenseite der Kanüle und der Innenseite der elastomeren Muffe ein Gleitmittel aufgetragen wird.

## Revendications

1. Appareil médical de perfusion, comprenant un récipient (3) de liquide à perfuser, un tube (4) allant du récipient au sujet destiné à recevoir la perfusion, une pompe (6) destinée à pomper le liquide de perfusion par le tube vers le sujet, et une soupape de sûreté (10) raccordée au tube entre la pompe et le sujet, la soupape de sûreté étant normalement fermée afin qu'elle empêche un écoulement libre du liquide depuis le récipient mais soit automatiquement ouverte lorsqu'une pression minimale prédéterminée est appliquée au liquide par la pompe, **caractérisé en ce que** la soupape de sûreté comporte une canule (20) ayant un passage axial (21) partant d'une première extrémité de la canule mais fermé à l'extrémité opposée, et un trou radial (23) adjacent à l'extrémité opposée et conduisant du passage axial vers la surface externe de la canule, et un manchon élastomère (24) placé autour de la canule et recouvrant le trou radial en le fermant normalement, la soupape de sûreté étant aussi manuellement déformable, par exemple par serrage, pliage ou torsion, afin que la soupape soit ouverte et permette l'amorçage de l'appareil par purge d'air.

2. Appareil selon la revendication 1, **caractérisé en ce que** la canule comporte un siège annulaire (26, 27) aux deux extrémités pour le logement du tube afin que les deux tronçons (11, 22) du tube soient raccordés l'un à l'autre lorsque la soupape de sûreté est placée entre eux.

3. Appareil selon la revendication 1, **caractérisé en ce que** la surface externe de l'extrémité opposée de la canule a un organe conformé à arête (25) destiné au positionnement du manchon élastomère.

4. Appareil selon la revendication 1, **caractérisé en ce qu'**un lubrifiant est appliqué entre la surface externe de la canule et la surface interne du manchon élastomère.
